# EUROPÄISCHE PATENTANMELDUNG

(11) **EP 2 742 981 A2**
(43) Veröffentlichungstag der Anmeldung: **18.06.2014**
(21) Anmeldenummer: 13193909.2
(22) Anmeldetag: 21.11.2013
(51) Int. Cl.: B01D 3/14, C07C 45/82

(54) **Aufarbeitung eines CDON/CDOL-Gemisches mittels einer "gelochten" Trennwandkolonne**

(30) Priorität: 17.12.2012 DE 102012223367
(71) Anmelder: Evonik Industries AG, 45128 Essen (DE)
(72) Erfinder: Cameretti, Luca, 44267 Dortmund (DE); Demicoli, Daniel, 45131 Essen (DE); Meier, Ralf, 44265 Dortmund (DE)

(57) **Zusammenfassung**

Die Erfindung betrifft ein Verfahren zur Abtrennung einer Cyclododecanon-reichen Fraktion aus einem Leichtsieder, Cyclododecanon, Mittelsieder, Cyclododecanol und Schwersieder enthaltenden Dehydrierungsgemisch, sowie einen Apparat zur Aufarbeitung eines solchen Dehydrierungsgemisches. Ihr liegt die Aufgabe zu Grunde, ein Aufarbeitungsverfahren für ein solches Gemisch anzugeben, bei dem eine Fraktion erhalten wird, die aus möglichst reinem Cyclododecanon besteht. Die Anlage zur Durchführung dieses Verfahrens soll dabei möglichst geringe Investitionskosten verursachen. Dies gelingt mit Hilfe einer "gelochten" Trennwandkolonne.

## Beschreibung

Die Erfindung betrifft ein Verfahren zur Abtrennung einer Cyclododecanon-reichen Fraktion aus einem Leichtsieder, Cyclododecanon, Mittelsieder, Cyclododecanol und Schwersieder enthaltenden Dehydrierungsgemisch.

Butadien wird im Folgenden als Kurzwort für die Substanz 1,3-Butadien, CAS-Nr. 106-99-0 verwendet.

CDT wird im Folgenden als Abkürzung verwendet für die Substanz 1,5,9-Cyclododecatrien, CAS-Nr. 4904-61-4.

CDEN wird im Folgenden als Abkürzung verwendet für die Substanz Cyclododecen, CAS-Nr. 1501-82-2.

CDAN wird im Folgenden als Abkürzung verwendet für die Substanz Cyclododecan, CAS-Nr. 294-62-2.

CDON im Folgenden als Abkürzung verwendet für die Substanz Cyclododecanon, CAS-Nr. 830-13-7.

CDOL wird im Folgenden als Abkürzung verwendet für die Substanz Cyclododecanol, CAS-Nr. 1724-39-6.

CDOL t.q. steht für CDOL in technischer Qualität und bezeichnet ein Gemisch enthaltend 75 bis 85 Gew.-% CDOL und 10 bis 20 Gew.-% CDON.

Oxim wird im Folgenden als Kurzwort gebraucht für das Oxim des CDON, CAS-Nr. 9466-89-4.

Laurinlactam ist der Trivialname von Azacyclotridecan-2-on, CAS-Nr. 947-04-6.

Laurinlactam ist der Ausgangsstoff für die Herstellung des Hochleistungskunststoffes Polyamid 12. Laurinlactam ist im industriellen Maßstab über die folgende Route erhältlich: Das bei der Erdölverarbeitung anfallende Butadien wird mittels katalytischer Cyclotrimerisierung in CDT umgesetzt. Durch Hydrierung des CDT entsteht CDAN. Die Oxidation des CDAN mit (Luft-) Sauerstoff ergibt ein Gemisch aus CDOL und CDON. Dieses Gemisch wird einer Dehydrierung unterworfen, welche das in dem Gemisch enthaltene CDOL in CDON umsetzt. Es fällt ein Dehydrierungsgemisch an, welches hauptsächlich CDON enthält. Daneben umfasst das Dehydrierungsgemisch nicht umgesetztes CDOL und weitere Komponenten. Hochreines CDON wird aus dem Dehydrierungsgemisch abgetrennt. Das hochreine CDON wird zu seinem Oxim oximiert. Das Oxim wird anschließend mit Schwefelsäure zu Laurinlactam umgesetzt.

Der gesamte Prozess ist mit weiteren Nachweisen beschrieben in Oenbrink, G. and Schiffer, T. 2009. Cyclododecanol, Cyclododecanone, and Laurolactam. Ullmann's Encyclopedia of Industrial Chemistry. DOI: 10.1002/14356007.a08_201.pub2

Die Erfindung befasst sich mit der Aufarbeitung des CDON / CDOL-haltigen Dehydrierungsgemisches mit dem Ziel, daraus hochreines CDON zu gewinnen.

Das auf der oben beschriebenen Route anfallende Dehydrierungsgemisch enthält neben CDON und CDOL weitere Komponenten in Gestalt von Leichtsiedern, Mittelsiedern und Schwersiedern.

"Leichtsieder" im Sinne dieser Erfindung sind Stoffe oder Stoffgemische, die bei denselben Druckbedingungen einen niedrigeren Siedepunkt als CDON aufweisen und sich daher bei der destillativen Trennung eines Gemisches aus Leichtsiedern und CDON im Destillat anreichern. Die wesentlichen Leichtsieder in diesem Zusammenhang sind: Cyclododecen (CDEN), Cyclododecan (CDAN), Dodecanal, Cyclododecan-Epoxid. Cyclododecan-Epoxid liegt auf der Grenze der obigen Definition der Leichtsieder, da sein Siedepunkt von etwa 150°C bei 40 mbar praktisch dem des CDON entspricht und daher von dem CDON nicht wirtschaftlich zu trennen ist. In geringeren Mengen (kleiner 100 ppm) kommen noch die Leichtsieder Essigsäure und Decan vor, diese sind aber für die Trennaufgaben kaum relevant.

"Mittelsieder" im Sinne dieser Erfindung sind Stoffe oder Stoffgemische, die bei denselben Druckbedingungen einen höheren Siedepunkt als CDON und einen niedrigeren Siedepunkt als CDOL aufweisen und sich daher bei der destillativen Trennung eines Gemisches aus CDON, Mittelsiedern und CDOL in der Mitte der Kolonne anreichern. Mittelsieder in diesem Zusammenhang ist insbesondere Dodecan-1-ol. Zur Fraktion der Mittelsieder gesellen sich weitere organische Substanzen, die bisher nicht genau charakterisiert werden konnten.

"Schwersieder" im Sinne dieser Erfindung sind Stoffe oder Stoffgemische, die bei denselben Druckbedingungen einen höheren Siedepunkt als CDOL aufweisen und daher bei der destillativen Trennung eines Gemisches aus Schwersiedern und CDOL im Rückstand verbleiben. Die Schwersiedergrenze liegt etwa bei 180°C bei einem Druck von 46 mbar. Zu den Schwersiedern gehört insbesondere Cyclododecandiol. Darüber hinaus enthält die Fraktion der Schwersieder weitere organische Substanzen, die bisher nicht näher charakterisiert werden konnten.

Die Dehydrierung von CDOL zu CDON ist beschrieben in den Dokumenten DE1568317 sowie in DE1248650. Dabei werden Dehydrierungsgemische beschrieben, die 74 bis 89 Gew.-% CDON und 25.9 bis 21.8 Gew.-% CDOL enthalten. Der Restanteil des hergestellten Dehydrierungsgemisches fällt auf Leichtsieder und Mittelsieder. Die Aufarbeitung des Dehydrierungsgemisches ist nicht weiter beschrieben.

Aus der japanischen Patentanmeldung JP05-000977A ist ein Verfahren zur Herstellung von hochreinem CDOL aus einem CDON/CDOL-Gemisch bekannt. Während der destillativen Aufarbeitung des Gemisches wird ein geringer Anteil an alkalischen Komponenten dem aufzutrennenden Gemisch zugegeben. Eine Trennwandkolonne wird zur Aufarbeitung des Gemisches nicht genutzt.

Die Oxidation von CDAN in ein Oxidationsgemisch enthaltend CDAN und CDOL ist in GB930842 beschrieben. Die nachfolgenden Verarbeitungsschritte sind nicht dargestellt.

In der DE2031782 ist ein Verfahren zur selektiven Herstellung von CDON beschrieben, bei dem CDAN oxidiert wird, um ein Gemisch aus CDON und CDOL zu erhalten. Das Gemisch wird destillativ aufgearbeitet, jedoch ohne nähere Beschreibung des Destillationsprozesses.

Aus der WO2009/092682 ist ein Verfahren zur Aufarbeitung eines CDON/CDOLhaltigen Gemisches bekannt, welches mit Hilfe einer Trennwandkolonne aufgearbeitet wird. Allerdings stellt bei diesem Verfahren der Mittelsieder die Hauptkomponente des Zulaufes dar, währenddessen die Leicht- bzw. die Schwersieder unerwünschte Nebenkomponenten darstellen.

Die Verarbeitung eines Laurinlactam-haltigen Gemisches in einer Trennwandkolonne ist in EP2336112A1 erwähnt. Auch hier besteht der Feed der Trennwandkolonne überwiegend aus Mittelsiedern.

Das für die Herstellung von Laurinlactam verwendete CDON sollte in einer möglichst reinen Form vorliegen, da Begleitkomponenten die Polymere im Polyamid 12 nachhaltig schädigen. Diese Nebenkomponenten entstehen insbesondere während der Oxidation des CDAN und auch während der Dehydrierung des CDOL.

Im Hinblick auf diesen Stand der Technik ist es Aufgabe der Erfindung, ein Verfahren zur Aufarbeitung eines Gemisches enthaltend Leichtsieder, CDON, Mittelsieder, CDOL und Schwersieder anzugeben, bei dem eine Fraktion erhalten wird, die aus möglichst reinem CDON besteht. Die Anlage zur Durchführung dieses Verfahrens soll möglichst geringe Investitionskosten verursachen.

Gelöst wird diese Aufgabe durch ein Verfahren mit den Merkmalen des Anspruch 1.

Gegenstand der Erfindung ist mithin ein Verfahren zur Abtrennung einer Cyclododecanon-reichen Fraktion aus einem Leichtsieder, Cyclododecanon, Mittelsieder, Cyclododecanol und Schwersieder enthaltenden Dehydrierungsgemisch, welches die folgenden Schritte umfasst:
a) Bereitstellen des Dehydrierungsgemisches;
b) destillatives Abtrennen der Leichtsieder aus dem Dehydrierungsgemisch unter Erhalt eines ersten Gemisches umfassend Cyclododecanon, Mittelsieder, Cyclododecanol und Schwersieder;
c) Einspeisen des ersten Gemisches in eine einen linken Kolonnenteil und einen rechten Kolonnenteil aufweisende Trennwandkolonne, bei welcher beide Kolonnenteile teilweise durch eine sich längs durch die Trennwandkolonne hindurch erstreckende Trennwand dergestalt getrennt sind, dass Kopf und Sumpf beider Kolonnenteile vereinigt sind, und bei welcher auf einer zwischen Kopf und Sumpf angeordneten Trennstufe eine die Trennwand umgehende stoffdurchlässige Verbindung beider Kolonnenteile vorgesehen ist;
d) Abziehen der Cyclododecanon-reichen Fraktion vom Kopf der Trennwandkolonne;
e) Abziehen eines zweiten Gemisches umfassend Cyclododecanon, Cyclododecanol und Mittelsieder aus einem Seitenabzug der Trennwandkolonne;
f) Abziehen einer Cyclododecanol und Schwersieder enthaltenden Fraktion vom Sumpf der Trennwandkolonne.

Kern der vorliegenden Erfindung ist die Verwendung einer "gelochten" Trennwandkolonne, welche eine stoffdurchlässige Verbindung zwischen beiden Kolonnenteilen aufweist. Der Begriff "Loch" ist in diesem Zusammenhang nicht so zu verstehen, dass die Trennwand denknotwendig eine Öffnung aufweist. Vielmehr ist der Begriff "Loch" im übertragenen Sinne zu verstehen als eine Verbindung zwischen beiden Kolonnenteilen, welche punktuell einen Stoffdurchtritt von einem zum anderen Kolonnenteil erlaubt, den die Trennwand sonst verhindert.

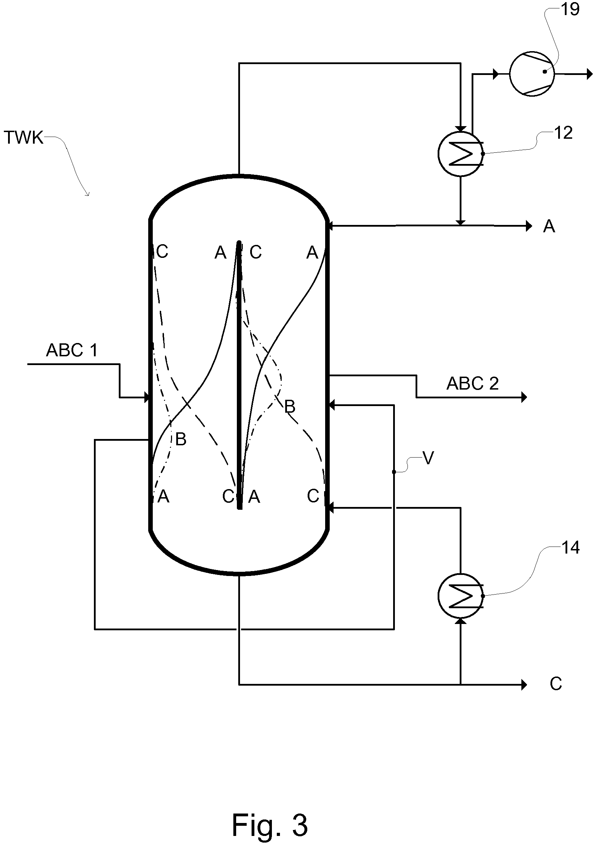

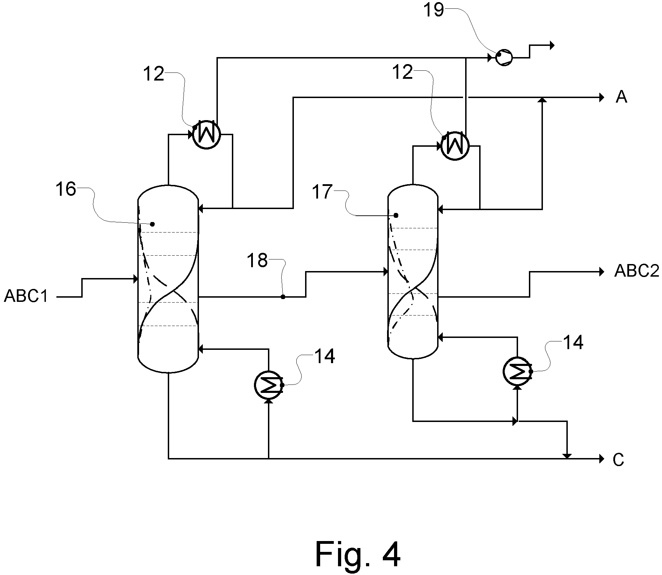

Aufgrund dieser Verbindung stellt die gelochte Trennwandkolonne eine bauliche Integration zweier hintereinander geschalteter Seitenabzug-Kolonnen mit vereinigtem Kopf und Sumpf dar. Vorteil dieser Kombination ist die Möglichkeit, insgesamt einen Kondensator und einen Verdampfer einzusparen, sodass die gelochte Trennwandkolonne geringere Investitionskosten verursacht als eine gleichwirkende Kombination zweier Einzel-Kolonnen. Der Vergleich zwischen der erfindungsgemäßen Trennwandkolonne mit "Loch" und einer trenntechnisch gleich wirkenden, jedoch kostspieligeren Anordnung zweier Seitenstromkolonnen mit vereinigtem Kopf- und Sumpfstrom wird noch anhand der Figuren 3 und 4 näher erläutert werden.

In einer bevorzugten Ausführungsform der Erfindung wird das erste Gemisch in den linken Kolonnenteil der Trennwandkolonne eingespeist und das zweite Gemisch auf dem rechten Kolonnenteil aus einem darin befindlichen Seitenabzug abgezogen. Mithin stellt die Verbindung eine Entnahme auf der Einspeiseseite und eine zusätzliche Einspeisung auf der Entnahmeseite dar.

In einer weiteren bevorzugten Ausführungsform der Erfindung ist die stoffdurchlässige Verbindung dafür eingerichtet, zwischen den beiden Kolonnenteilen flüssige Mittelsieder zu transportieren. Die Verbindung stellt in diesem Fall eine Flüssigkeitsleitung zwischen den beiden Kolonnenteilen dar.

Um sicher zu stellen, dass ausschließlich flüssige Stoffe über die stoffdurchlässige Verbindung die Kolonnenseite wechseln, sollte die Verbindung an einem Flüssigkeitssammler im linken Kolonnenteil entspringen. Flüssigkeitssammler sind in der Destillationstechnik gemeinhin bekannt und dienen in erster Linie dazu, die von einem Packungs- oder Schüttungsbett herabrieselnde Flüssigkeit zu sammeln und auf einen Flüssigkeitsverteiler zu geben, der die Flüssigkeit auf ein darunter liegendes Bett gleichmäßig verteilt. Optional kann ein Teil der gesammelten Flüssigkeit als Seitenabzug entnommen werden. Die Verbindung zwischen den beiden Kolonnenteilen wird also in herkömmlicher Weise als Seitenstrom links abgezogen und rechts wie ein Feed aufgegeben. Die Vorgabe, dass ausschließlich ein flüssiger Stoffstrom zwischen den beiden Kolonnenteilen ausgetauscht wird, ist auch ein Grund dafür, dass die erfindungsgemäße Trennwandkolonne nicht denknotwendig ein Loch in der Trennwand aufweist, da ein einfaches Loch auch für gasförmige Medien durchlässig ist.

In einer ganz besonders bevorzugten Ausführungsform der Erfindung wird die stoffdurchlässige Verbindung so positioniert, dass sie auf der Trennebene des linken Kolonnenteils entspringt, auf welcher die flüssige Konzentration im Mittelsieder in dem linken Kolonnenteil maximal ist, und dass die stoffdurchlässige Verbindung auf der Trennebene des rechten Kolonnenteils mündet, auf welcher die Zusammensetzung der flüssigen Phase möglichst derjenigen des von dem linken Kolonnenteil entnommenen, flüssigen Stroms entspricht. Dies bedeutet, dass im linken Teil der Trennwandkolonne der Mittelsieder auf wenige Prozente aufkonzentriert wird, am Konzentrationsbauch angezapft und dieser Strom über die Verbindung auf der rechten Seite wieder eingespeist wird. Auf der rechten Seite kann dann die Kolonne mit einem erhöhten Rücklaufverhältnis betrieben werden, sodass der Mittelsieder auf hohe Werte von bis zu 40 % aufkonzentriert und im Seitenstrom entnommen werden kann.

Vorzugsweise wird die Trennwandkolonne unter Vakuum gefahren; also bei einem Druck unter 1 bar absolut. Insbesondere sollte der Druck innerhalb der Trennwandkolonne unter 50 mbar absolut liegen.

Das Durchmesserverhältnis vom rechten Kolonnenteil zum linken Kolonnenteil sowie die Anzahl etwaiger Einbauten in den beiden Kolonnenteilen sollten so gewählt werden, dass sich unter Berücksichtigung der Äquivalenz des hydraulischen Widerstandes für Gase in beiden Kolonnenteilen die gewünschten Strömungsverhältnisse einstellen.

Vorzugsweise dient das erfindungsgemäße Verfahren dazu, ein Dehydrierungsgemisch aufzuarbeiten, welches die folgende, sich zu 100 % ergänzende Zusammensetzung aufweist:

| | |
|---|---|
| Leichtsieder (LB): | 1 bis 8 Gew-%, bevorzugt 3 Gew-%; |
| Cyclododecanon (CDON): | 60 bis 90 Gew-%, bevorzugt 70 Gew-%; |
| Mittelsieder (MB): | 0 bis 1.5 Gew-%, bevorzugt 1 Gew-%; |
| Cyclododecanol (CDOL): | 10 bis 40 Gew-%, bevorzugt 24 Gew-%; |
| Schwersieder (HB): | 0.1 bis 2.5 Gew-%, bevorzugt 2 Gew-%. |

Das Verfahren dient vorzugsweise dazu, eine Cyclododecanon-reiche Fraktion zu erhalten, die einen besonders hohen Reinheitsgrad aufweist. Erfindungsgemäß sollte diese Fraktion einen CDON-Gehalt von mindestens 98 Gew.-% aufweisen, bevorzugt wird sogar ein CDON-Gehalt von 99,5 Gew.-% angestrebt. Des Weiteren sollte die am Kopf der Trennwandkolonne abgezogene Cyclododecanon-reiche Fraktion möglichst frei von CDOL, Schwersiedern und Mittelsiedern sein. Die am Sumpf der Trennwandkolonne abgezogene Fraktion sollte demgegenüber möglichst frei von CDON sein.

Sofern das erfindungsgemäße Verfahren im Zuge eines Laurinlactam-Prozesses eingesetzt wird, umfasst der Arbeitsschritt "Bereitstellen des Dehydrierungsgemisches" die folgenden Teilschritte:
g) Oxidation von Cyclododecan mit Sauerstoff unter Erhalt eines Oxdiationsgemisches enthaltend Leichtsieder, Cyclododecanon, Mittelsieder, Cyclododecanol und Hochsieder;
h) destillatives Abtrennen einer Cyclododecanol-reichen Fraktion aus dem Oxidationsgemisch, welches hinsichtlich Schwersieder abgereichert ist;
i) Dehydrierung der Cyclododecanol-reichen Fraktion unter Erhalt des Dehydrierungsgemisches.

Sofern das erfindungsgemäße Verfahren Teil eines Laurinlactam-Prozesses ist, empfiehlt es sich, den Sumpf der Trennwandkolonne in den Schritt h) zurückzuführen. Diese Sumpffraktion enthält nämlich zu einem großen Teil CDOL, das auf diese Weise dem Dehydrierungsprozess wieder zugängig gemacht und in CDON umgesetzt werden kann. Die Schwersieder konzentrieren sich bis zu einer Grenzkonzentration auf und werden mit dieser im Kreis geführt. Die über die Oxidation in den Prozess neu eingetragenen Schwersieder verlassen den Prozess wieder über den Sumpf der Vorabscheiderkolonne und über den Seitenabzug der Trennwandkolonne.

Das Kopfprodukt der Trennwandkolonne stellt hochreines CDON dar, welches sich hervorragend dazu eignet, oximiert und sodann zu Laurinlactam weiter verarbeitet zu werden. Das zweite von der Trennwandkolonne gelieferte Gemisch kann entweder weiter aufgearbeitet werden, um reines CDON und/oder CDOL zu erhalten (beispielsweise durch Batch-Destillation) oder es wird thermisch zu verwertet. Letztes geschieht einfachstenfalls durch Verbrennung. Die fühlbare Wärme kann ggf. vorher noch abgegriffen werden.

Gegenstand der Erfindung ist auch die im Verfahren eingesetzte Trennwandkolonne als solche. Bei dieser handelt es sich um einen Apparat für die destillative Aufarbeitung von Stoffgemischen in drei Fraktionen umfassend einen von einer Hülle umschlossenen Raum, welcher aufgeteilt ist in einen linken Kolonnenteil, einen rechten Kolonnenteil, einen Sumpf und einen Kopf, bei welchem sich innerhalb der Hülle, zwischen Sumpf und Kopf, durch die Trennwandkolonne hindurch eine Trennwand erstreckt, welche den linken Kolonnenteil von dem rechten Kolonnenteil trennt und bei welchem ein Zulauf für die Aufgabe des Stoffgemisches, ein Abzug am Kopf für den Abzug der ersten Fraktion, einen Abzug am Sumpf für den Abzug der zweiten Fraktion und einen Seitenabzug für den Abzug der dritten Fraktion vorgesehen ist, und bei welchem zwischen Kopf und Sumpf eine stoffdurchlässige Verbindung zwischen den beiden Kolonnenteilen angeordnet ist. Der erfindungsgemäße Apparat weist somit einen vereinten Kopf und einen vereinten Sumpf auf.

Die stoffdurchlässige Verbindung zwischen den beiden Kolonnenteilen ist vorzugsweise außerhalb der Hülle des Apparats angeordnet. Die Verbindung stellt bevorzugt einen Abzug von der linken Seite und einen Zulauf für die rechte Seite dar. Weitere vorteilhafte Merkmale dieses Apparats gehen aus der Beschreibung und den Beispielen hervor.

Ein weiterer Gegenstand der Erfindung ist die Verwendung dieses Apparats zur Aufarbeitung eines Stoffgemisches enthaltend Leichtsieder, CDON, Mittelsieder, CDOL und Schwersieder.

Die Erfindung soll nun anhand von Ausführungsbeispielen näher erläutert werden. Hierfür zeigen:
- Figur 1:: Verfahrensfließbild;
- Figur 2:: Detailansicht Trennwandkolonne;
- Figur 3:: Konzentrationsprofile innerhalb der Trennwandkolonne;
- Figur 4:: Ersatzschaubild der Trennwandkolonne.

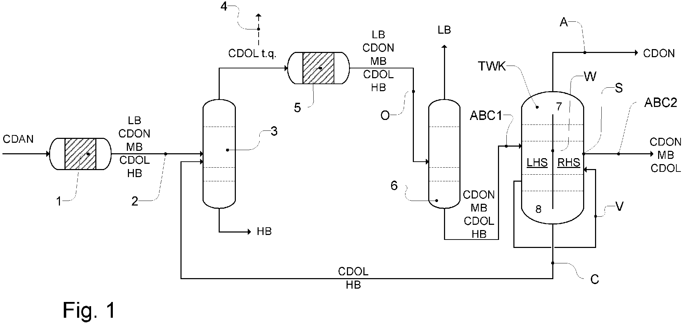

Der gesamte Aufarbeitungsprozess ist in Figur 1 dargestellt. Er beginnt in einer Oxidation 1, in welcher CDAN mit Sauerstoff oxidiert wird. Dabei fällt ein Oxidationsgemisch 2 an, welches Leichtsieder LB, CDON, Mittelsieder MB, CDOL und Hochsieder HB enthält. Aufgrund des Reaktionsmechanismus ist der Gehalt an CDON innerhalb des Oxidationsgemisches 2 deutlich geringer als der des CDOL. Typischerweise enthält ein derartiges Oxidationsgemisch 2 etwa 15 Gew.-% CDON und etwa 70 Gew.-% CDOL.

Das Oxidationsgemisch 2 wird in eine Vorabscheiderkolonne 3 eingespeist. Die Funktion dieser Vorabscheiderkolonne 3 besteht darin, einen großen Anteil der Hochsieder HB auszuschleusen. Dies geschieht über den Sumpf. Über den Kopf der Vorabscheiderkolonne 3 wird CDOL t.q. abgezogen. CDOL t.q. enthält etwa 84 Gew.-% CDOL und 13 Gew.-% CDON. CDOL t.q. stellt ein separates Verkaufsprodukt dar und kann gegebenenfalls über einen Abzweig 4 aus dem Prozess ausgeschleust werden.

Zu der Laurinlactamherstellung wird CDOL t.q. einer Dehydrierung 5 unterworfen. Dabei wird CDOL zu CDON dehydriert, sodass sich der Anteil dieser beiden Stoffe umkehrt. Das aus der Dehydrierung 5 abgezogene Dehydrierungsgemisch O weist typischerweise die folgende, sich zu 100% ergänzende Zusammensetzung auf:

| | |
|---|---|
| Leichtsieder (LB): | 1 bis 8 Gew-%, bevorzugt 3 Gew-%; |
| Cyclododecanon (CDON): | 60 bis 90 Gew-%, bevorzugt 70 Gew-%; |
| Mittelsieder (MB): | 0 bis 1.5 Gew-%, bevorzugt 1 Gew-%; |
| Cyclododecanol (CDOL): | 10 bis 40 Gew-%, bevorzugt 24 Gew-%; |
| Schwersieder (HB): | 0.1 bis 2.5 Gew-%, bevorzugt 2 Gew-%. |

Das Dehydrierungsgemisch O wird nun in eine Leichtsiederkolonne 6 eingespeist. Zweck der Leichtsiederkolonne 6 ist es, die Leichtsieder LB über Kopf destillativ aus dem Dehydrierungsgemisch O abzutrennen, sodass am Sumpf der Leichtsiederkolonne 6 ein erstes Gemisch ABC1 umfassend CDON, Mittelsieder MB, CDOL und Schwersieder HB anfällt. Die Leichtsieder LB werden in diesem Schritt vorzugsweise vollständig abgetrennt. Die Besonderheit des Sumpfproduktes ABC1 besteht darin, dass sein Gehalt an Mittelsiedern MB äußerst gering ist, nämlich lediglich etwa 1 Gew.-% beträgt. Im Übrigen besteht das Gemisch ABC1 im Wesentlichen aus CDON, CDOL und Hochsiedern HB. Eine typische Zusammensetzung des Gemisches ABC1 ist wie folgt:

| | |
|---|---|
| Leichtsieder (LB): | 0 bis 1 Gew-%, bevorzugt 0 Gew-%; |
| Cyclododecanon (CDON): | 60 bis 90 Gew-%, bevorzugt 70 Gew-%; |
| Mittelsieder (MB): | 0 bis 2 Gew-%, bevorzugt 1 Gew-%; |
| Cyclododecanol (CDOL): | 10 bis 40 Gew-%, bevorzugt 26 Gew-%; |
| Schwersieder (HB): | 0.1 bis 3 Gew-%, bevorzugt 3 Gew-%. |

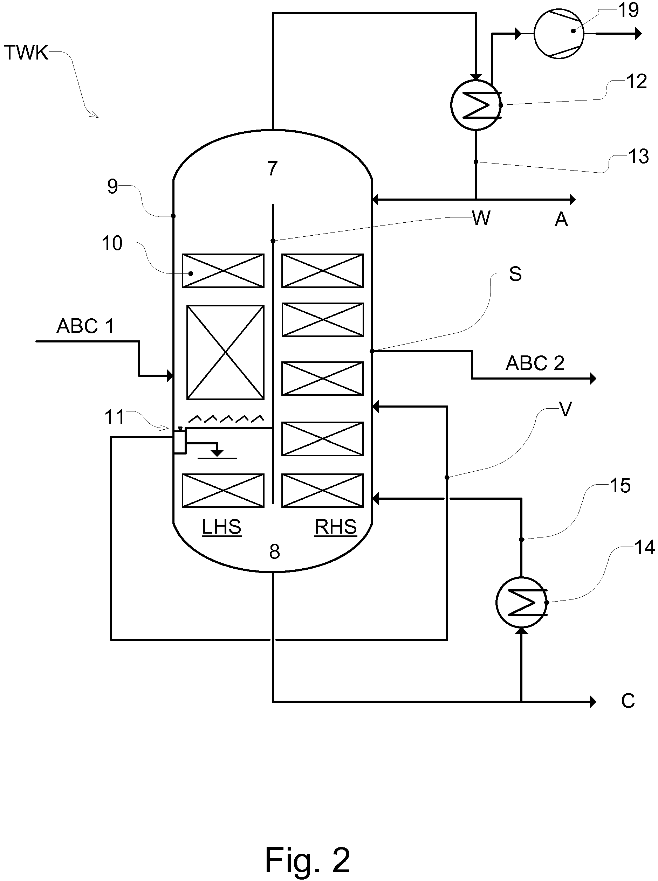

Gemisch ABC1 wird sodann in eine Trennwandkolonne TWK eingespeist. Die Trennwandkolonne TWK umfasst eine Trennwand W, welche einen linken Teil der Trennwandkolonne LHS von einem rechten Teil der Trennwandkolonne RHS abtrennt. Kopf 7 und Sumpf 8 der Trennwandkolonne sind vereint. In einer Trennebene zwischen Kopf 7 und Sumpf 8 befindet sich eine Verbindung V zwischen den beiden Hälften der Trennwandkolonne LHS und RHS. Die Verbindung V stellt somit einen Abzug von der linken Seite und einen Zulauf für die rechte Seite dar. An der rechten Seite der Trennwandkolonne RHS ist auch ein Seitenabzug S vorgesehen. Der Aufbau der Trennwandkolonne TWK geht aus der Figur 2 genauer hervor.

Vom Kopf 7 der Trennwandkolonne TWK wird eine CDON-reiche Fraktion A abgezogen, die zu 99,5 % aus CDON besteht. Vom Sumpf 8 der Trennwandkolonne TWK wird eine Fraktion C abgezogen, die CDOL und Schwersieder HB enthält. Diese Sumpffraktion C wird recycliert und zusammen mit dem Oxidationsgemisch 2 in die Vorabscheiderkolonne 3 geführt. Auf diese Weise wird das nicht umgesetzte CDOL wieder der Dehydrierung 5 zugeführt. Die zurückgeführten Hochsieder HB konzentrieren sich in dem Prozess zu einem bestimmten Maße auf. Die aus der Oxidation 1 frisch herein getragenen Hochsieder HB werden über den Sumpf der Vorabscheiderkolonne 3 ausgeschieden, sodass sich hinsichtlich der Hochsieder-Konzentration ein stationärer Zustand einstellt.

Über den Seitenabzug S wird ein zweites Gemisch ABC2 abgezogen, welches CDON, CDOL und Mittelsieder enthält. Die Mittelsieder MB stellen dabei den größten Anteil des Seitengemisches ABC2 dar. Der Anteil der Wertprodukte CDON und CDOL ist geringer. Gemisch ABC2 kann beispielsweise durch Batch-Destillation weiter aufgereinigt oder einer thermischen Verwertung zugeführt werden. Das vom Kopf 7 der Trennwandkolonne TWK abgezogene, hochreine CDON der Fraktion A wird zur Laurinlactamherstellung verwendet (nicht dargestellt).

Der schematische Aufbau der Trennwandkolonne TWK ist in Figur 2 näher dargestellt. Die Trennwandkolonne TWK umfasst eine Hülle 9, die einen inneren Raum umschließt. Der Raum gliedert sich auf in einen linken Kolonnenteil LHS, einen rechten Kolonnenteil RHS, einen Kopf 7 und einen Sumpf 8. Innerhalb der Hülle 9 erstreckt sich durch die Trennwandkolonne TWK eine Wand W, welche den linken Kolonnenteil vom rechten Kolonnenteil stofflich trennt. Im Kopf 7 und am Sumpf 8 sind beide Kolonnenteile stofflich vereint, da sich die Trennwand W nicht über die gesamte Höhe der Trennwandkolonne TWK erstreckt. Kopf 7 und Sumpf 8 beginnen dort, wo Trennwand W aufhört.

In die Trennwandkolonne TWK können eine Vielzahl von an sich bekannter Einbauten 10 eingebaut sein, wie beispielsweise Packungen oder Füllkörper der Firma Sulzer oder der Firma Montz. Zweck der Einbauten 10 ist es, eine möglichst große Anzahl von theoretischen Trennstufen zu erreichen.

Des Weiteren können in die Trennwandkolonne TWK verschiedene Flüssigkeitssammler und -verteiler 11 vorgesehen sein, baulich ausgeführt in fachüblicher Weise.

Wesentliches Merkmal der erfindungsgemäß eingesetzten Trennwandkolonne TWK ist eine stoffdurchlässige Verbindung V von der linken Kolonnenseite LHS zur rechten Kolonnenseite RHS. Bei der Verbindung V handelt es sich um eine Flüssigkeitsleitung, die zur Herstellung einer Strömung durch die Leitung mit einer nicht dargestellten Zirkulationspumpe versehen ist. Mittels der Verbindung V wird flüssiger Mittelsieder von dessen Konzentrationsmaximum auf der linken Seite zur rechten Seite der Kolonne transferiert; siehe hierzu auch Erläuterung zur Figur 4. Um sicher zu stellen, dass über die Verbindung V ausschließlich flüssige Stoffe ausgetauscht werden, entspringt die Verbindung V von einem Flüssigkeitssammler 11 auf der linken Seite. Der Flüssigkeitssammler 11 ist auf einer Trennebene eingebaut, an welcher die flüssige Konzentration der Mittelsieder ein Maximum aufweist. Die stoffdurchlässige Verbindung entspringt damit auf der Trennebene des linken Kolonnenteils, auf welcher die flüssige Konzentration im Mittelsieder in dem linken Kolonnenteil maximal ist. Die stoffdurchlässige Verbindung V mündet auf der Trennebene des rechten Kolonnenteils, auf welcher die Zusammensetzung der flüssigen Phase möglichst derjenigen des von dem linken Kolonnenteil entnommenen, flüssigen Stroms entspricht. Dies bedeutet, dass im linken Teil der Trennwandkolonne der Mittelsieder auf wenige Prozente aufkonzentriert wird, am Konzentrationsbauch angezapft und dieser Strom über die Verbindung auf der rechten Seite wieder eingespeist wird. Abzugs- und Einspeisepunkt der Verbindung V werden demnach den Konzentrationsverhältnissen entsprechend gewählt und müssen nicht auf derselben Ebene liegen.

Der über die Verbindung V transportierte Stoffstrom weist die folgende, sich zu 100 % ergänzende Zusammensetzung auf:

| | |
|---|---|
| Cyclododecanon (CDON): | 30 bis 50 Gew-%, bevorzugt 43 Gew-%; |
| Mittelsieder (MB): | 0 bis 20 Gew-%, bevorzugt 18 Gew-%; |
| Cyclododecanol (CDOL): | 30 bis 50 Gew-%, bevorzugt 38.4 Gew-%; |
| Schwersieder (HB): | 0.1 bis 2 Gew-%, bevorzugt 0.6 Gew-%. |

Vom Kopf 7 der Trennwandkolonne wird ein gasförmiges Kopfprodukt A abgezogen, welches fast ausschließlich CDON (ca. 99.5 Gew.%) enthält. Wie bei Destillationskolonnen üblich, sind am Kopfabzug ein Kondensator 12 und ein Kopfrücklauf 13 vorgesehen. Am Kopf ist auch ein Vakuumsystem 19 angeordnet, welches in der Trennwandkolonne TWK einen Unterdruck aufbaut.

Am Sumpf 8 der Trennwandkolonne TWK wird eine Fraktion C abgezogen, die überwiegend CDOL sowie Hochsieder enthält. Wie bei Destillationskolonnen üblich, ist hier ein Sumpferhitzer/Verdampfer 14 mit dem entsprechenden Sumpfrücklauf 15 vorgesehen.

An der rechten Seite der Trennwandkolonne RHS ist ein Seitenabzug S vorgesehen, über welchem ein an Mittelsiedern reiches, zweites Gemisch ABC2 abgezogen wird. Das aus dem Seitenabzug abgezogene Gemisch ABC2 enthält im Wesentlichen CDON, CDOL und Mittelsieder.

Der über den Seitenabzug entnommene Stoffstrom ABC2 weist die folgende, sich zu 100 % ergänzende Zusammensetzung auf:

| | |
|---|---|
| Cyclododecanon (CDON): | 20 bis 50 Gew-%, bevorzugt 35 Gew-%; |
| Mittelsieder (MB): | 0 bis 60 Gew-%, bevorzugt 54 Gew-%; |
| Cyclododecanol (CDOL): | 10 bis 50 Gew-%, bevorzugt 10 Gew-%; |
| Schwersieder (HB): | 0 bis 2 Gew-%, bevorzugt 1 Gew-%. |

In Figur 3 ist die Trennwandkolonne abermals aufgezeichnet, nun aber mit den Konzentrationsprofilen CDON (durchgezogene Linie, Fraktion A), CDOL (gestrichelte Linie, Fraktion C) und Mittelsieder (strichpunktierte Linie, Fraktion B). Die Verbindung V ist so angeordnet, dass sie auf der Trennebene des linken Kolonnenteils entspringt, auf welcher die flüssige Konzentration im Mittelsieder in dem linken Kolonnenteil maximal ist. Die Verbindung mündet auf der Trennebene des rechten Kolonnenteils, auf welcher die Zusammensetzung der flüssigen Phase möglichst derjenigen des von dem linken Kolonnenteil entnommenen, flüssigen Stroms entspricht. Dies bedeutet, dass im linken Teil der Trennwandkolonne der Mittelsieder auf wenige Prozente aufkonzentriert wird, am Konzentrationsbauch angezapft und dieser Strom über die Verbindung auf der rechten Seite wieder eingespeist wird. Auf diese Weise wird in der linken Kolonne das Gemisch ABC1 so getrennt, dass CDON als reines Destillat und CDOL als reiner Schwersieder gewonnen werden. Der Mittelsieder bildet innerhalb des linken Teils der Trennwandkolonne einen Konzentrationsbauch aus. An der Stelle des Konzentrationsmaximums wird ein Teilstrom flüssigen Mittelsieders entnommen und über die Verbindung V in den rechten Teil der Trennwandkolonne überführt. Dort wird mit erhöhtem Rücklauf erneut eine scharfe Trennung von CDON und CDOL durchgeführt. Dabei wird der Mittelsieder wiederum innerhalb des rechten Teils der Tennwandkolonne stark aufkonzentriert und kann über den Seitenstrom S abgeführt werden. Da die Destillate beider Kolonnenteile und die Sümpfe beider Kolonnenteile jeweils die gleiche Konzentration aufweisen (Reinprodukte) können sie zusammengeführt und über jeweils ein Kondensationssystem 12, ein Vakuumsystem 19 und einen Verdampfer 14 gefahren werden.

Eine trenntechnisch gleichwertige Lösung ist in Figur 4 dargestellt: Hier wird nicht mit einer Trennwandkolonne gearbeitet, sondern es werden zwei herkömmliche Seitenabzug-Kolonnen 16 und 17 hintereinander verschaltet. Die erste Kolonne 16 entspricht dem linken Teil der Trennwandkolonne, der zweite Teil 17 den rechten Teil der Trennwandkolonne. Die Funktion der Verbindung V übernimmt ein Seitenstrom 18 zwischen der ersten Kolonne 16 und der zweiten Kolonne 17. Sümpfe und Destillate beider Kolonnen 17 und 18 werden zu gemeinschaftlichen Fraktionen A und C vereinigt. Die Konzentrationsprofile in beiden Kolonnen 16 und 17 werden so gewählt wie im linken bzw. im rechten Teil der Trennwandkolonne (vergleiche Figur 3). Auf diese Weise wird dasselbe Trennergebnis erreicht. Allerdings erfordert die in Figur 4 dargestellte Anlage größere Investitionskosten, da beide Kolonnen 16 und 17 jeweils einen individuellen Verdampfer 14 und Kondensator 12 benötigen. Da die in Figur 3 dargestellte Trennwandkolonne jeweils nur einen Verdampfer und einen Kondensator benötigt, ist die in Figur 3 dargestellte, erfindungsgemäße Ausführung deutlich kostengünstiger.

### Bezugszeichenliste

- 1: Oxidation
- 2: Oxidationsgemisch
- 3: Vorabscheiderkolonne
- 4: Abzweig
- 5: Dehydrierung
- 6: Leichtsiederkolonne
- 7: Kopf Trennwandkolonne
- 8: Sumpf Trennwandkolonne
- 9: Hülle
- 10: Einbauten Kolonne
- 11: Flüssigkeitssammler
- 12: Kondensator
- 13: Kopfrücklauf
- 14: Sumpferhitzer / Verdampfer
- 15: Sumpfrücklauf
- 16: erste Kolonne
- 17: zweite Kolonne
- 18: Seitenstrom
- 19: Vakuumsystem
- LB: Leichsieder
- MB: Mittelsieder
- HB: Schwersieder
- TWK: Trennwandkolonne
- RHS: rechter Teil der Trennwandkolonne
- LHS: linker Teil der Trennwandkolonne
- W: Trennwand
- V: Verbindung
- S: Seitenabzug
- O: Dehydrierungsgemisch
- A: CDON-reiche Fraktion (Kopf Trennwandkolonne)
- B: Mittelsieder reiche Fraktion (Verbindung Trennwandkolonnenseiten)
- C: CDON/Hochsieder-haltige Fraktion (Sumpf Trennwandkolonne)
- ABC1: erstes Gemisch (Feed Trennwandkolonne)
- ABC2: zweites Gemisch (Seitenabzug Trennwandkolonne)

## Patentansprüche

1. Verfahren zur Abtrennung einer Cyclododecanon-reichen Fraktion (A) aus einem Leichtsieder (LB), Cyclododecanon (CDON), Mittelsieder (MB), Cyclododecanol (CDOL) und Schwersieder (HB) enthaltenden Dehydrierungsgemisch (O), **gekennzeichnet durch** die folgenden Schritte:
a) Bereitstellen des Dehydrierungsgemisches (O);
b) destillatives Abtrennen der Leichtsieder (LB) aus dem Dehydrierungsgemisch (O) unter Erhalt eines ersten Gemisches (ABC1) umfassend Cyclododecanon (CDON), Mittelsieder (MB), Cyclododecanol (CDOL) und Schwersieder (HB);
c) Einspeisen des ersten Gemisches (ABC1) in eine einen linken Kolonnenteil (LHS) und einen rechten Kolonnenteil (RHS) aufweisende Trennwandkolonne (TWK), bei welcher beide Kolonnenteile (LHS, RHS) teilweise **durch** eine sich längs **durch** die Trennwandkolonne (TWK) hindurch erstreckende Trennwand (W) dergestalt getrennt sind, dass Kopf (7) und Sumpf (8) beider Kolonnenteile (LHS, RHS) vereinigt sind, und bei welcher auf einer zwischen Kopf (7) und Sumpf (8) angeordneten Trennstufe eine die Trennwand (W) umgehende stoffdurchlässige Verbindung (V) beider Kolonnenteile (LHS, RHS) vorgesehen ist;
d) Abziehen der Cyclododecanon-reichen Fraktion (A) vom Kopf (7) der Trennwandkolonne (TWK);
e) Abziehen eines zweiten Gemisches (ABC2) umfassend Cyclododecanon (CDON), Cyclododecanol (CDOL) und Mittelsieder (MB) aus einem Seitenabzug (S) der Trennwandkolonne (TWK);
f) Abziehen einer der Cyclododecanol (CDOL) und Schwersieder (HB) enthaltenden Fraktion (C) vom Sumpf (8) der Trennwandkolonne (TWK).

2. Verfahren nach Anspruch 1, **dadurch gekennzeichnet, dass** das erste Gemisch (ABC1) in den linken Kolonnenteil (LHS) eingespeist wird, und dass das zweite Gemisch (ABC2) aus dem sich am rechten Kolonnenteil (RHS) befindlichen der Seitenabzug (S) abgezogen wird.

3. Verfahren nach Anspruch 2, **dadurch gekennzeichnet, dass** die stoffdurchlässige Verbindung (V) zwischen den beiden Kolonnenteilen (LHS, RHS) für den Transport flüssiger Mittelsieder (MB) vom linken Kolonnenteil (LHS) zum rechten Kolonnenteil (RHS) eingerichtet ist.

4. Verfahren nach Anspruch 3, **dadurch gekennzeichnet, dass** die stoffdurchlässige Verbindung (V) an einem Flüssigkeitssammler (11) im linken Kolonnenteil (LHS) entspringt.

5. Verfahren nach Anspruch 3 oder 4, **dadurch gekennzeichnet, dass** die stoffdurchlässige Verbindung (V) auf der Trennebene des linken Kolonnenteils (LHS) entspringt, auf welcher die flüssige Konzentration der Mittelsieder (MB) in dem linken Kolonnenteil (LHS) maximal ist, und dass die stoffdurchlässige Verbindung (V) auf der Trennebene des rechten Kolonnenteils (RHS) mündet, auf welcher die Zusammensetzung des Stoffstroms aus dem linken Kolonnenteil (LHS) möglichst dem der flüssigen Phase auf der Trennebene des rechten Kolonnenteils (RHS) entspricht.

6. Verfahren nach einem der Ansprüche 1 bis 5, **dadurch gekennzeichnet, dass** die Trennwandkolonne (TWK) bei einem Druck von unter 1 bar absolut, insbesondere unter 50 mbar absolut betrieben wird.

7. Verfahren nach einem der Ansprüche 1 bis 6, **dadurch gekennzeichnet, dass** das Dehydrierungsgemisch (O) die folgende, sich zu 100 % ergänzende Zusammensetzung aufweist:
| | |
|---|---|
| Leichtsieder (LB): | 1 bis 8 Gew-%, bevorzugt 3 Gew-%; |
| Cyclododecanon (CDON): | 60 bis 90 Gew-%, bevorzugt 70 Gew-%; |
| Mittelsieder (MB): | 0 bis 1.5 Gew-%, bevorzugt 1 Gew-%; |
| Cyclododecanol (CDOL): | 10 bis 40 Gew-%, bevorzugt 24 Gew-%; |
| Schwersieder (HB): | 0.1 bis 2.5 Gew-%, bevorzugt 2 Gew-%. |

8. Verfahren nach einem der Ansprüche 1 bis 7, **dadurch gekennzeichnet, dass** die Cyclododecanon-reiche Fraktion (A) einen Gehalt an Cyclododecanon (CDON) von mindestens 98 Gew.-%, bevorzugt von 99.5 Gew.-% aufweist, und/oder dass die Cyclododecanon-reiche Fraktion (A) frei von Cyclododecanol (CDOL), Schwersiedern (HB) und Mittelsiedern (MB) ist.

9. Verfahren nach einem der Ansprüche 1 bis 8, **dadurch gekennzeichnet, dass** die Cyclododecanol (CDOL) und Schwersieder (HB) enthaltende Fraktion (C) frei von Cyclododecanon (CDON) ist.

10. Verfahren nach einem der Ansprüche 1 bis 9, **dadurch gekennzeichnet, dass** das Bereitstellen des Dehydrierungsgemisches (O) die folgenden Schritte umfasst:
g) Oxidation von Cyclododecan (CDAN) mit Sauerstoff unter Erhalt eines Oxdiationsgemisches (2) enthaltend Leichtsieder (LB), Cyclododecanon (CDON), Mittelsieder (MB), Cyclododecanol (CDOL) und Hochsieder (HB);
h) destillatives Abtrennen einer Cyclododecanol-reichen Fraktion (CDOL t.q.) aus dem Oxidationsgemisch (2), welche hinsichtlich Schwersieder (HB) abgereichert ist;
i) Dehydrierung der Cyclododecanol-reichen Fraktion (CDOL t.q.) unter Erhalt des Dehydrierungsgemisches (O).

11. Verfahren nach Anspruch 10, **dadurch gekennzeichnet, dass** die Cyclododecanol (CDOL) und Schwersieder (HB) enthaltenden Fraktion (C) in den Schritt h) zurückgeführt wird.

12. Verfahren nach einem der Ansprüche 1 bis 11, **dadurch gekennzeichnet,**
**dass** die Cyclododecanon-reiche Fraktion (A) zu Laurinlactam weiterverarbeitet wird und/oder dass das zweite Gemisch (ABC2) entweder weiter aufgetrennt oder thermisch verwertet wird.

13. Apparat für die destillative Aufarbeitung von Stoffgemischen in drei Fraktionen, genannt Trennwandkolonne (TWK), umfassend einen von einer Hülle (9) umschlossenen Raum, welcher aufgeteilt ist einen linken Kolonnenteil (LHS), einen rechten Kolonnenteil (RHS), einen Sumpf (8) und einen Kopf (7), bei welchem sich innerhalb der Hülle (9), zwischen Sumpf (8) und Kopf (7), durch die Trennwandkolonne (TWK) hindurch eine Trennwand (W) erstreckt, welche den linken Kolonnenteil (LHS) von dem rechten Kolonnenteil (RHS) trennt, und bei welchem ein Zulauf für die Aufgabe des Stoffgemisches (ABC1), ein Abzug am Kopf für den Abzug der ersten Fraktion (A), einen Abzug am Sumpf für den Abzug der zweiten Fraktion (C) und ein Seitenabzug (S) für den Abzug der dritten Fraktion (ABC2) vorgesehen ist,
**gekennzeichnet durch**
eine zwischen Kopf (7) und Sumpf (8) angeordnete, stoffdurchlässige Verbindung (V) zwischen beiden Kolonnenteilen (LHS, RHS).

14. Verwendung eines Apparats gemäß Anspruch 13 zur Aufarbeitung eines Stoffgemisches enthaltend Leichtsieder (LB), Cyclododecanon (CDON), Mittelsieder (MB), Cyclododecanol (CDOL) und Schwersieder (HB).
